# EUROPEAN PATENT SPECIFICATION

(11) **EP 0 458 437 B1**
(45) Date of publication and mention of the grant of the patent: **09.10.1996**
(21) Application number: 91301386.8
(22) Date of filing: 21.02.1991
(51) Int. Cl.: C12N 15/12, C12P 21/02, C12N 15/71, C12N 1/11

(54) **Process for production of human nerve growth factor by genetic engineering**
Verfahren zur Herstellung von menschlichem Nervenwachstumsfaktor durch Genmanipulation
Procédé pour la production du facteur humain de croissance des nerfs par génie génétique

(30) Priority: 21.02.1990 JP 38358/90
(43) Date of publication of application: 27.11.1991
(73) Proprietor: HITACHI, LTD., Chiyoda-ku, Tokyo 100 (JP)
(72) Inventor: Shimizu, Norio, Sayama-shi (JP); Fujimori, Kiyoshi, Shiki-shi (JP); Fukuzono, Shinichi, Hatoyamamachi, Hiki-gun Saitama-ken (JP); Kotomura, Naoe, Kawagoe-shi (JP)
(74) Representative: Baillie, Iain Cameron

(56) References cited:
- EP-A- 121 338
- EP-A- 329 175
- EP-A- 335 400
- CHEMICAL ABSTRACTS, vol. 100, no. 17, 23 April 1984, Columbus, OH (US); V.R. LINGAPPA et al., p. 150, no. 100:1334450

## Description

### BACKGROUND OF THE INVENTION

### 1. Field of the Invention

The present invention relates to synthetic gene DNA encoding human nerve growth factor (hereinafter simply referred to as "hNGF"), a plasmid vector carrying the gene, a transformant transformed with the plasmid and processes for production of hNGF using the transformant.

### 2. Related Art Statement

Nerve growth factor (NGF) is known as a polypeptide which participates in survival of nerve cells, extension of neuraxon or differentiation of nerve cells. NGF extracted from the submaxillary gland of matured male mouse is a polypeptide having a molecular weight of 140,000 and is called 7SNGF in view of its sedimentation constant. In this NGF, ß subunit is a polypeptide which independently exhibits NGF activity by itself and is composed of 118 amino acids; this NGF is termed ßNGF. On the other hand, with regard to human NGF, any tissue as in the submaxillary gland of mouse where NGF is present in large quantities is not noted. Therefore, using ßNGF cDNA of mouse as a probe, human gene library was screened to isolate hNGF gene and the amino acid sequence of hNGF was determined (Japanese Patent Application Laid-Open No. 60-84299).

hNGF is considered to be effective as a drug for the treatment of neuropathy. For this purpose, hNGF is required in large quantities. On the other hand, it has been made possible to produce a foreign protein in E. coli in large quantities by genetic engineering technique. Attempts have also been made on hNGF to produce the same by E. coli using genetic engineering technique. For example, in Japanese Patent Application Laid-Open No. 60-84299, expression of a gene has been attempted using natural hNGF gene. This technique comprises incorporating hNGF gene into an expression vector and culturing cells, especially, E. coli, bearing the vector to produce hNGF in large quantities.

In the prior art techniques described above, however, sufficient consideration has not been made to arrange the DNA sequence encoding hNGF for achieving a high expression efficiency, to enhance an expression efficiency of the vector, and to modify medium conditions for the transformants to those suitable for the production of hNGF, etc. Therefore, the prior art techniques involve problems that it was difficult to readily produce hNGF by host cells, especially by E. coli, in large quantities.

### SUMMARY OF THE INVENTION

An object of the present invention is to provide a newly synthesized hNGF gene DNA having a high expression efficiency in host cells such as E. coli, etc., and a recombinant plasmid constructed by incorporating said DNA together with a promoter having a high expression efficiency, as well as a transformed microorganism having an excellent hNGF productivity which is obtained by transforming with this recombinant plasmid. Another object of the present invention is to provide a novel process for production of hNGF efficiently, by applying the optimum medium conditions.

As a result of extensive investigations to solve the foregoing problems, the present inventors have accomplished the present invention. The characteristic features of the present invention reside in (1) through (11) described below.
(1) DNA encoding human nerve growth factor and having the following nucleotide sequence: 
A G C T C T T C C C A C C C G A T T T T C
C A C C G T G G C G A A T T C T C C G T G
T G T G A C T C T G T C T C T G T A T G G
G T A G G C G A T A A A A C C A C T G C C
A C T G A T A T C A A A G G C A A A G A G
G T G A T G G T G C T G G G C G A A G T T
A A C A T T A A C A A C T C T G T A T T C
A A A C A G T A C T T C T T C G A A A C C
A A A T G C C G T G A C C C A A A C C C G
G T T G A C T C T G G C T G C C G C G G C
A T C G A T T C T A A A C A C T G G A A C
T C T T A C T G C A C C A C T A C T C A C
A C C T T C G T A A A A G C T T T G A C T
A T G G A T G G T A A A C A G G C T G C C
T G G C G T T T C A T C C G T A T C G A T
A C T G C A T G C G T G T G T G T A C T G
T C C C G T A A A G C T G T T C G T
 wherein DNA encoding the signal sequence of β-lactamase is ligated upstream of the 5' end of said DNA encoding human nerve growth factor.
(2) DNA according to (1), wherein said DNA encoding the signal sequence of β-lactamase has the following nucleotide sequence: 
A T G A G T A T T C A A C A T T T C C G T
G T C G C C C T T A T T C C C T T T T T T
G C G G C A T T T T G C C T T C C T G T T
T T T G C G
(3) DNA according to (1) or (2), wherein further tryptophan regulatory gene is ligated upstream of said signal sequence.
(4) DNA encoding human nerve growth factor and having the following nucleotide sequence: 
A G C T C T T C C C A C C C G A T T T C
C A C C G T G G C G A A T T C T C C G T G
T G T G A C T C T G T C T C T G T A T G G
G T A G G C G A T A A A A C C A C T G C C
A C T G A T A T C A A A G G C A A A G A G
G T G A T G G T G C T G G G C G A A G T T
A A C A T T A A C A A C T C T G T A T T C
A A A C A G T A C T T C T T C G A A A C C
A A A T G C C G T G A C C C A A A C C C G
G T T G A C T C T G G C T G C C G C G G C
 
A T C G A T T C T A A A C A C T G G A A C
T C T T A C T G C A C C A C T A C T C A C
A C C T T C G T A A A A G C T T T G A C T
A T G G A T G G T A A A C A G G C T G C C
T G G C G T T T C A T C C G T A T C G A T
A C T G C A T G C G T G T G T G T A C T G
T C C C G T A A A G C T G T T C G T
 wherein DNA encoding the N terminus of trpL polypeptide in tryptophan operon ligated with a tryptophan regulatory gene is ligated upstream of the 5' end of said DNA encoding human nerve growth factor.
(5) DNA according to (4), wherein said DNA encoding the N terminus of trpL polypeptide in tryptophan operon has the following nucleotide sequence: 
A T G A A A G C A A T T T T C G T G G A A
T T C
(6) A plasmid vector carrying DNA described in any one of (1) to (5).
(7) A transformant obtained by transforming a microorganism with a plasmid vector described in (6).
(8) A transformant described in (7), wherein the microorganism is E. coli.
(9) A process for producing human nerve growth factor which comprises culturing a transformant described in (7) or (8) to produce human nerve growth factor.
(10) A process for producing human nerve growth factor described in (9), wherein the transformant is cultured in the presence of 3-β-indolylacrylic acid (IA) as an expression inducer.
(11) A process for producing human nerve growth factor described in (9) or (10), wherein the transformant is cultured in medium free of tryptophan.

### BRIEF DESCRIPTION OF THE DRAWINGS

Fig. 1 shows a synthetic DNA sequence A containing the signal sequence and the former half amino acids of hNGF;
Fig. 2 shows a synthetic DNA sequence B containing the latter half amino acids of hNGF;
Fig. 3 shows 8 DNA fragments of A-1 through A-8 in the DNA sequence A;
Fig. 4 shows 6 DNA fragments of B-1 through B-6 in the DNA sequence B;
Fig. 5 shows restriction enzyme maps of the DNA sequences A and B;
Fig. 6 shows the construction of plasmid pTRSNGFA;
Fig. 7 shows the construction of plasmid pTRLNGFB;
Fig. 8 shows the construction of plasmid pTRSNGF;
Fig. 9 shows the construction of plasmid pTRLNGF;

### DETAILED DESCRIPTION OF THE PREFERRED EMBODIMENTS

Hereafter the present invention is described in detail.

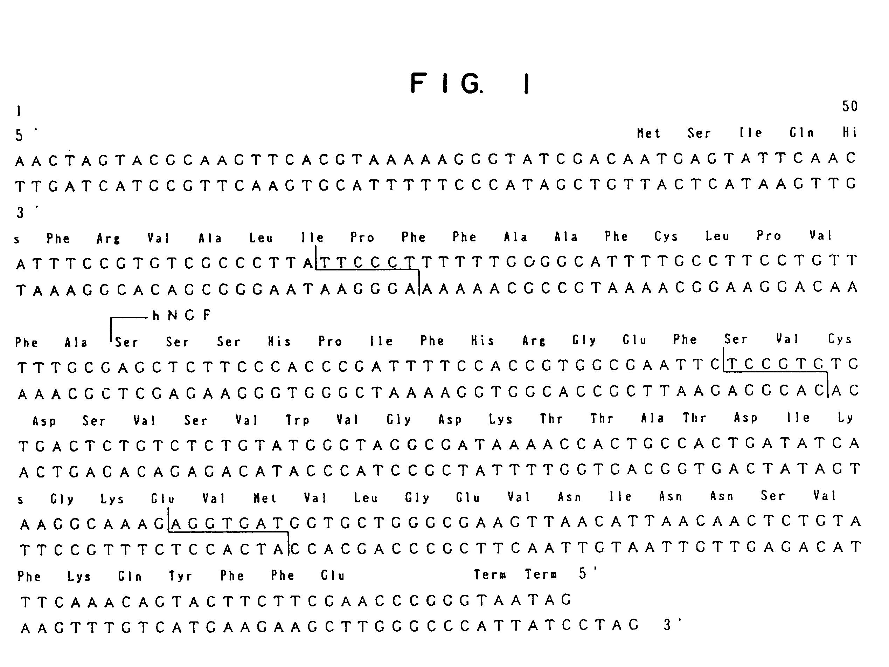

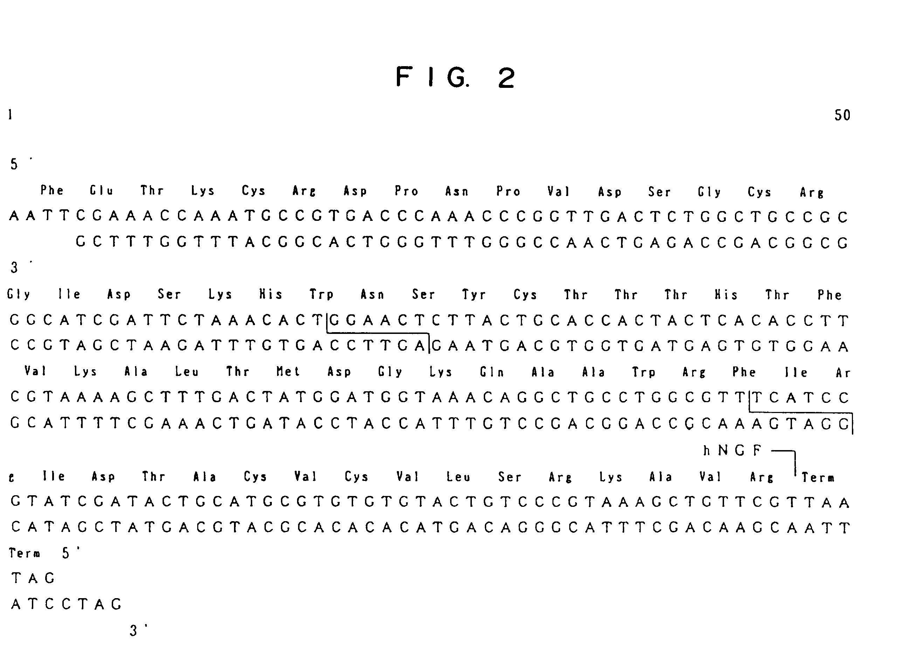

The amino acid sequence of hNGF was previously identified by Ullrich et al. (Nature, 303, 821 (1983)) and is composed of 118 amino acids. The nucleotide sequence of hNGF gene may be apparently deduced from the correspondence table of the amino acids to genetic codons. However, since there are a plurality of genetic codons corresponding to one amino acid, this correspondence is not primarily determined. In order to obtain hNGF gene DNA having a high expression efficiency, consideration should be taken as to whether, among several nucleotide sequences deduced from the amino acid sequence of hNGF, genetic codons used in the nucleotide sequences would appear with a high frequency in host cells, especially in E. coli, as to whether the structure would readily cause an error in organic synthesis, and further as to whether mRNA transcribed from DNA would easily form a secondary structure so that efficiency of translation in ribosome might be decreased. In the present invention, these points have been taken into consideration and the nucleotide sequence shown in the characteristic feature (1) of the present invention has been designed, since the sequence is considered to be most pertinent as the nucleotide sequence of the structural gene of hNGF. There are then constructed two DNA strands shown by DNA sequence A in Fig. 1 and shown by DNA sequence B in Fig. 2, which strands contain the former half sequence and the latter half sequence of the nucleotide sequence of the structural gene of hNGF, respectively. These DNAs are independently introduced into plasmid pTRLBT1. E. coli strain HB101 carrying pTRLBT1 has been deposited as HB101 (pTRLBT1) in the Fermentation Research Institute of the Agency of Industrial Science & Technology of Japan and given Accession Number FERM P-9907, and then transferred to an international deposition under the Budapest Treaty and given Accession Number BP-02348. Using the two recombinant plasmids, the nucleotide sequence of full length hNGF gene, in which DNA sequence A has been ligated with DNA sequence B, is prepared.

DNA sequence A in Fig. 1 is described more specifically. The DNA sequence is composed of 283 base pairs of DNA prepared from 8 DNA fragments synthesized by organic chemistry technique using, e.g., an automated DNA synthesizer (Applied Biosystems Inc., Model 380B). The DNA sequence has restriction enzyme Hpa I site at the 5′ end and BamH I site at the 3′ end and comprises a part of tryptophan regulatory gene (promoter), a nucleotide sequence of signal peptide of β-lactamase, a gene encoding amino acids from serine at the N-terminal to glutamic acid at 55-position in the amino acid sequence of hNGF, and termination codons, TAA and TAG. In addition, it is designed so that Sma I site (CCCGGG) is provided before the termination codons and β-galactosidase gene may be ligated therewith utilizing the Sma I site.

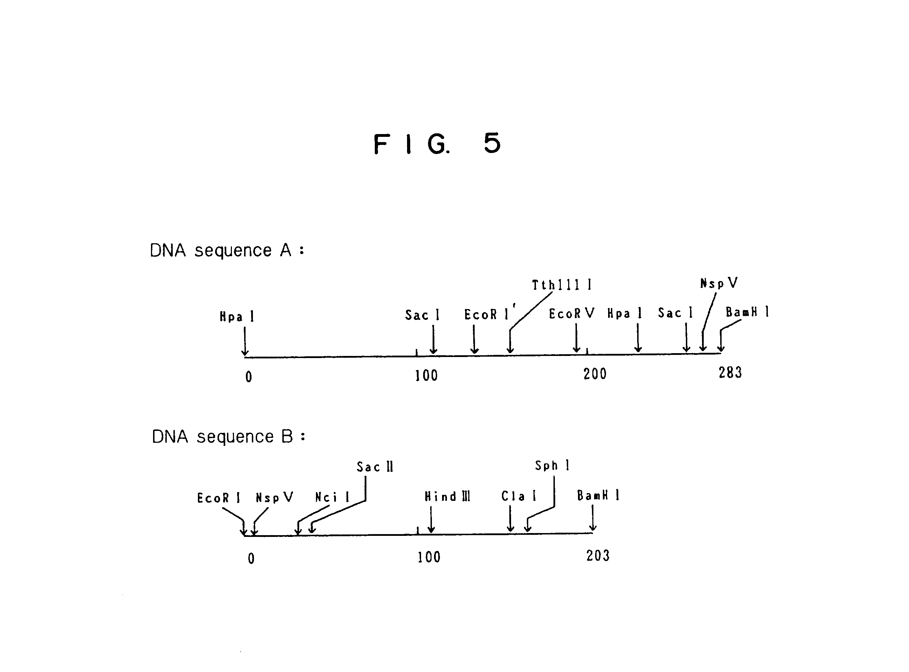

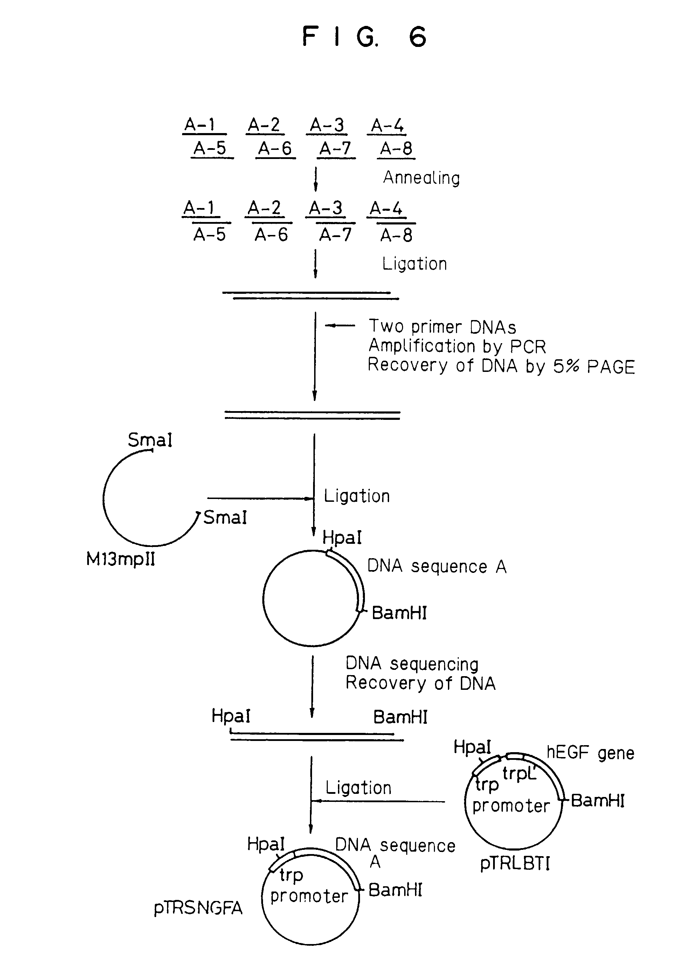

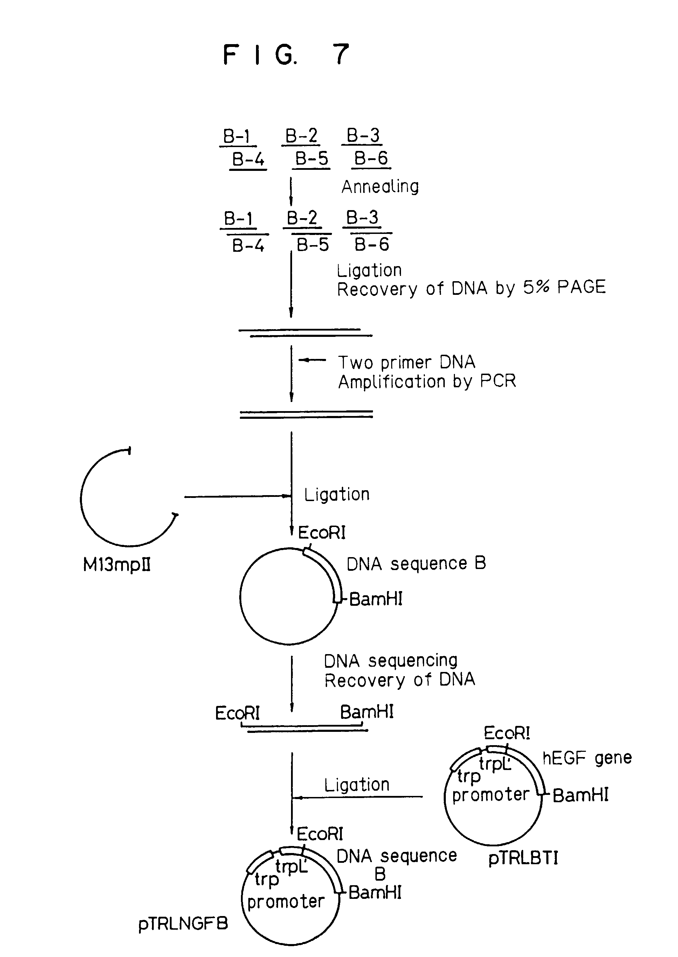

DNA sequence B in Fig. 2 is composed of 203 base pairs prepared from 6 DNA fragments similarly synthesized by organic chemistry technique. The DNA sequence has restriction enzyme Eco RI site at the 5′ end and BamH I site at the 3′ end and comprises a gene encoding amino acids from phenylalanine at 54-position to arginine at 118-position in the amino acid sequence of hNGF, and termination codons, TAA and TAG. Restriction enzyme maps of these DNA sequences A and B are shown in Fig. 5. DNA sequences A and B are inserted between Hpa I site and Bam HI site and between Eco RI site and Bam HI site of plasmid vector pTRLBT1 to give plasmids pTRSNGFA in Fig. 6 and pTRLNGFB in Fig. 7, respectively, which enables the cloning of DNA sequences A and B.

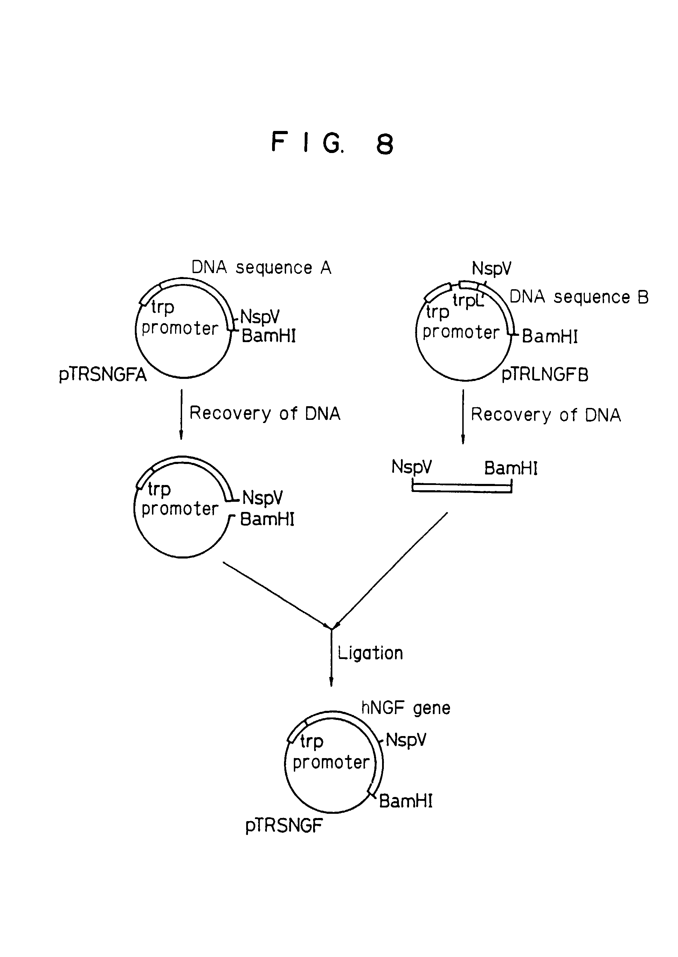

In this case, a part of tryptophan promoter in DNA sequence A at the plasmid pTRSNGFA in Fig. 6 has the nucleotide sequence corresponding to the latter half of tryptophan promoter. By ligating the part of tryptophan promoter with the former half of tryptophan promoter in the Hpa I/Bam HI DNA fragment of pTRLBT1, tryptophan promoter is reconstructed. As stated above, cloned DNA sequence A or B can be expressed independently in E. coli and can be obtained as peptide A or B as a divided hNGF protein. The full length hNGF gene may be prepared by ligation utilizing restriction enzyme Nsp V site (TTCGAA) at the 3′ end of DNA sequence A and the same restriction enzyme Nsp V site at the 5′ end of DNA sequence B (cf. Fig. 8). The plasmid bearing the thus prepared hNGF gene has a good expression efficiency because it has a tryptophan promoter derived from pTRSNGFA. Furthermore, the plasmid has the signal sequence of β-lactamase upstream of the hNGF gene and hence, matured hNGF can be secreted into the periplasmic space of E. coli by culturing recombinant E. coli carrying the plasmid.

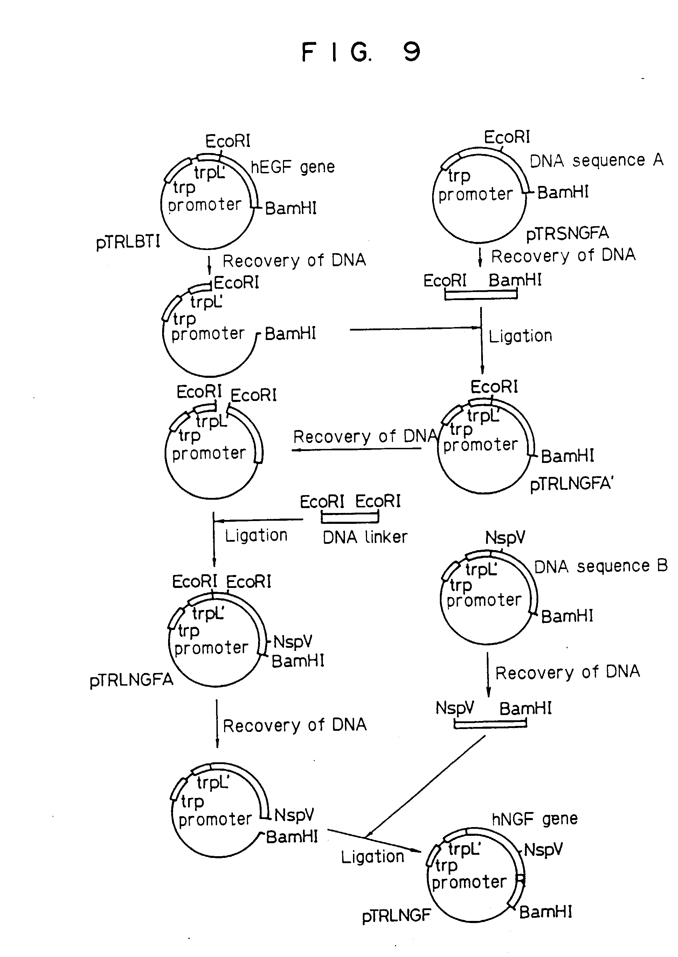

Further as another process for preparing the plasmid bearing the full length hNGF gene DNA of the present invention, the following process can be given (cf. Fig. 9).

That is, the fragment obtained by digesting the plasmid pTRSNGFA having DNA sequence A with Eco RI and Bam HI is ligated with the fragment obtained by digesting plasmid pTRLBT1 similarly with Eco RI and Bam HI to construct a plasmid pTRLNGFA′. After a linker is ligated with the plasmid pTRLNGFA′ having DNA sequence A at the Eco RI site to construct a plasmid pTRLNGFA, the plasmid is digested with Nsp V and Bam HI to prepare Nsp V/Bam HI DNA fragment. This fragment is ligated with a fragment obtained by digesting the aforesaid plasmid pTRLNGFB having DNA sequence B with Nsp V and Bam HI thereby to ligate DNA sequence A with DNA sequence B. Thus, a plasmid pTRLNGF having the nucleotide sequence of the full length hNGF gene can be obtained. This plasmid has upstream of the 5′ end of the full length hNGF gene a DNA sequence encoding the N terminus of trpL polypeptide in tryptophan operon which is ligated with tryptophan promoter. The DNA sequence which is shown by trpL' in Fig. 9 and which encodes the N terminus of this trpL polypeptide has the following nucleotide sequence.

A T G A A A G C A A T T T T C G T G G A A T T C

Therefore, the polypeptide obtained by culturing a microorganism transformed with the plasmid pTRLNGF is in the fused form of 8 amino acids peptide derived from this trpL' and hNGF polypeptide.

The thus obtained plasmids pTRLNGF can efficiently express hNGF gene.

Further by transforming a host microorganism such as E. coli, etc. with these plasmids pTRSNGF, and pTRSNGF each bearing the full length hNGF gene encoding 118 amino acids of hNGF polypeptide, a transformed microorganism capable of producing hNGF can be obtained.

When this transformed microorganism is cultured to produce hNGF, it is preferred that 3-β-indolylacrylic acid (IA), which is analogous to tryptophan, be supplemented to medium, in order to initiate expression of the gene. It is also preferred to use tryptophan-free medium. Thus, the production yield of hNGF can be further improved. This is due to the following reasons.

That is, expression of a gene is regulated by a promoter. In the case of trp promoter, RNA polymerase binds to trp promoter and this enzyme shifts to a site downstream of the promoter to transcribe DNA nucleotide sequence, whereby RNA is synthesized. Ribosome translates information on the synthesized RNA to synthesize a polypeptide. On the other hand, when the concentration of tryptophan in a cell increases, tryptophan binds to repressor to activate the repressor and the repressor binds to an operator region. As the result, there is no chance that RNA polymerase binds to the promoter region, so that RNA is not synthesized and production of the polypeptide stops. In this case, tryptophan acts as an inhibitor. Therefore, the present inventors have adopted the process of initiating expression of the gene, by inactivating repressor by the addition of IA or changing to tryptophan-free medium, utilizing regulation system of genetic expression in trp promoter.

In the present invention, the nucleotide sequence of hNGF gene is designed to be a nucleotide sequence having a high expression efficiency in a host microorganism, especially in E. coli and at the same time, the gene is incorporated into a plasmid, together with a promoter having an excellent expression efficiency. Thus, the microorganism transformed with the plasmid produces the hNGF with an extremely good expression efficiency. In addition, medium conditions are further improved so that hNGF can be produced easily in large quantities. Therefore, the present invention greatly contributes to production of drugs.

Hereafter the present invention is described more specifically by referring to the examples but is not intended to be limited thereto.

### Example 1

### (1) Design of the nucleotide sequence of hNGF structural gene and synthesis of small DNA fragment by organic chemistry technique

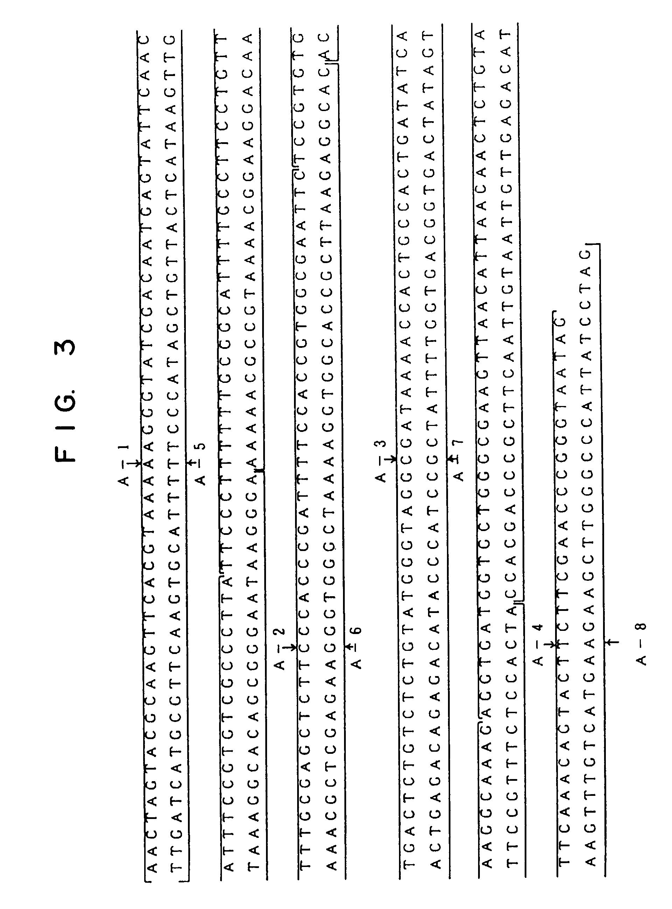

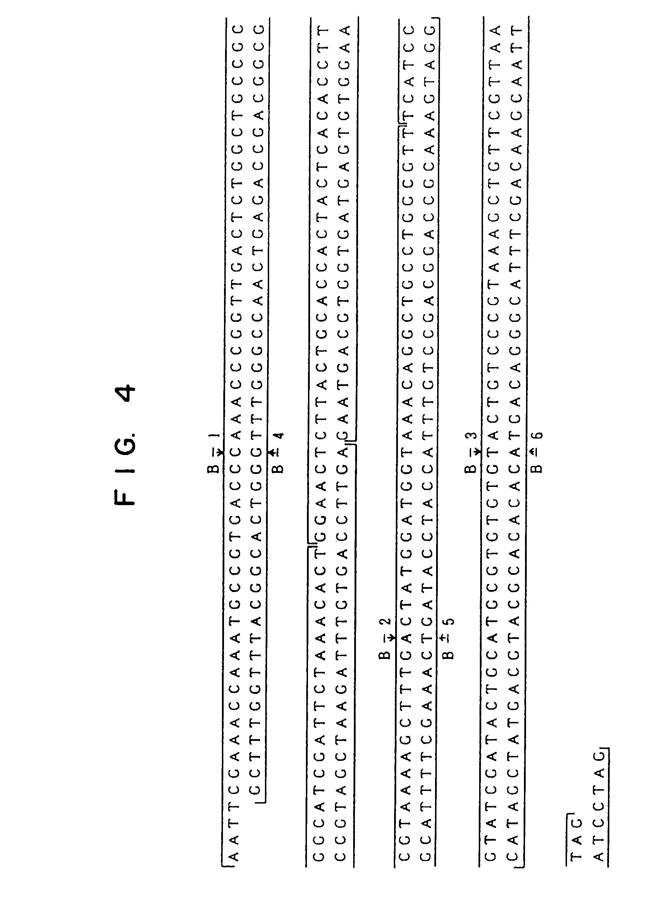

By reference to the table of appearance frequency of codon in bacteria (Couy et al., Nucleic Acids Res., 10, 7055 (1982)), the nucleotide sequence of hNGF was designed in such a way that genetic codons with a high appearance frequency in E. coli were selected for each amino acid and various restriction enzyme sites were introduced within the hNGF structural gene to replace genes in a simple manner. Based on this design, 8 single stranded DNAs of A-1 through A-8 (Fig. 3) and 6 single stranded DNAs of B-1 through B-6 (Fig. 4) were synthesized using an automated DNA synthesizer (Applied Biosystems Inc., Model 380B). These single stranded DNAs are small fragments having base pairs of 57 to 75.

Each of the synthesized single stranded DNAs was cut out of resin carrier and then subjected to electrophoresis on 8% polyacrylamide gel modified with urea. The gel fragment containing a DNA band having the desired size was cut out and DNA was recovered by electrophoresis. After the recovered DNA was concentrated, DNA was purified by phenol extraction and ethanol precipitation and then dissolved in sterile water. By the foregoing procedures, 100 to 200 µg of the desired single stranded DNAs could be obtained.

### (2) Preparation of a plasmid bearing DNA sequence A containing the former half nucleotide sequence of the hNGF gene (cf. Fig. 6)

Using 1 µl (10 U) of T4 polynucleotide kinase and 2 µl of 10 mM ATP, 250 pmols each of the six DNA fragments of A-2 through A-7 chemically synthesized were reacted at 37°C for an hour to phosphorylate the 5′ end. Using 50 pmols each of the DNA fragments, A-1 and A-5, A-2 and A-6, A-3 and A-7, and A-4 and A-8 were annealed as follows. After 50 µl of mineral oil was added to 8 µl of the reaction solution, the mixture was kept at 75°C for 10 minutes and then cooled over 4.5 hours to prepare 4 double stranded DNAs. The prepared 4 double stranded DNAs were mixed with each other and ligated using a ligation kit (manufactured by Takara Shuzo Co., Ltd.). After 0.5 µl (2.5 U) of Taq DNA polymerase, 16 µl (1.25 mM) of 4 kinds of dNTP, 5 µl each of two primers (DNA obtained by adding GTT to DNA fragment A-1 at the 5′ end followed by phosphorylation and DNA obtained by adding G to DNA fragment A-8 at the 5′ end followed by phosphorylation) were added, buffer was further added to make the whole volume 100 µl, and 100 µl of mineral oil was also added to effect amplification by PCR (polymerase chain reaction). The reaction was repeated 25 times under conditions at 94°C for a minute, at 55°C for 2 minutes and at 72°C for 3 minutes and then the reaction was finally carried out under conditions at 72°C for 17 minutes. Then, mineral oil was removed and ethanol precipitation treatment was conducted twice. The DNA mixture produced by PCR was subjected to 5% polyacrylamide gel electrophoresis and the gel fragment containing a DNA band having the desired size was cut out. DNA was recovered by electrophoresis. After the recovered DNA was concentrated, DNA was purified by phenol extraction and ethanol precipitation and then dissolved in sterile water. To 1 µl of the purified DNA solution was added 1 µl of M13 mp11 phage DNA previously digested with Sma I. After ligation using a ligation kit, E. coli strain JM103 was transformed. Twenty two plaques having the inserted fragment were obtained. DNA sequencing reveals that 3 out of 22 plaques contained the desired DNA sequence A. M13 mp11 phage DNA having the desired DNA sequence A was digested with Bam HI and Hpa I to recover DNA sequence A of 283 base pairs. DNA sequence A was ligated with pTRLBT1 previously digested with Bam HI and Hpa I similarly. E. coli strain C600 was transformed with the ligation product, whereby 8 colonies were obtained. The DNAs of 8 clones were digested with restriction enzyme for analysis. The results reveal that two clones contained the desired plasmid. Thus, plasmid pTRSNGFA (32 µg) having DNA sequence A shown by the nucleotide sequence of Fig. 1 could be obtained.

The restriction enzyme map of DNA sequence A is shown in Fig. 5.

### (3) Preparation of a plasmid bearing DNA sequence B having the latter half nucleotide sequence of the hNGF gene (cf. Fig. 6)

Using 1 µl (10 U) of T4 polynucleotide kinase and 10 µl of 10 mM ATP, 300 pmols each of the 4 DNA fragments of B-2 through B-5 chemically synthesized were reacted in the whole volume of 100 µl, which was made with buffer, to phosphorylate the 5′ end. After B-1 and B-4, B-2 and B-5 and B-3 and B-6 were kept at 70°C for 10 minutes, each of the mixtures was then cooled over 4.5 hours to effect annealing, whereby 3 double stranded DNAs were prepared. The prepared 3 double stranded DNAs were mixed using 30.5 µl of double strand of B-1 and B-4, 57 µl of double strand of B-2 and B-5 and, 39.3 µl of double strand of B-3 and B-6. After phenol extraction and ethanol precipitation, they were ligated with each other using the ligation kit. The ligated DNA was subjected to 5% polyacrylamide gel electrophoresis and the gel fragment containing a DNA band having the desired size was cut out. DNA was recovered by electrophoresis. After the recovered DNA was concentrated, DNA was purified by phenol extraction and ethanol precipitation and then dissolved in sterile water. To 1 µl of a solution containing about the half of the purified DNA were added 0.5 µl (2.5 U) of Taq DNA polymerase, 16 µl (1.25 mM) of 4 kinds of dNTP, two primers, i.e., 2.5 µl of DNA obtained by phosphorylation of DNA fragment B-1 at the 5′ end and 3.8 µl of DNA obtained by phosphorylation of DNA fragment B-6 at the 5′ end. Buffer was further added to make the whole volume 100 µl and, 100 µl of mineral oil was also added to perform PCR. The reaction was repeated 25 times under conditions at 94°C for a minute, at 55°C for 2 minutes and at 72°C for 3 minutes and then the reaction was finally carried out under conditions at 72°C for 7 minutes. Then, mineral oil was removed and ethanol precipitation treatment was conducted twice. After 1 µl of M13 mp11 phage DNA digested with Xba I and then filled up was added to 1 µl of the DNA aqueous solution produced by PCR, they were ligated using a ligation kit. Then, E. coli strain JM103 was transformed. Five plaques having the inserted fragment were obtained. DNA sequencing reveals that 1 out of 5 plaques had the desired DNA sequence B. M13 mp11 phage DNA having the desired DNA sequence B was digested with Eco RI and Bam HI to recover DNA sequence B of 203 base pairs. DNA sequence B was ligated with pTRLBT1 previously digested with Eco RI and Bam HI similarly. E. coli strain C600 was transformed with the ligation product, whereby 170 colonies were obtained. Among them, DNAs of 16 clones were digested with restriction enzyme for analysis. The results reveal that all of the clones contained the desired plasmid. Thus, 152 µg of plasmid pTRLNGFB having DNA sequence B shown by the nucleotide sequence of Fig. 2 could be obtained.

The restriction enzyme map of DNA sequence B is shown in Fig. 5.

### (4) Preparation of a plasmid containing the full length hNGF gene (cf. Fig. 8)

After 1 µg of plasmid pTRSNGFA was digested with 20 U of Nsp (7524) V and 20 U of Bam HI, electrophoresis was performed on 0.8% agarose gel to recover the DNA fragment containing the vector and DNA sequence A. On the other hand, 7 µg of plasmid pTRLNGFB was digested with 20 U of Nsp (7524) V and 40 U of Bam HI, electrophoresis was performed on 5% polyacrylamide gel to recover the DNA fragment containing DNA sequence B of 199 bp. After 1 µl each of these two DNA fragments were ligated using the ligation kit, E. coli strain C600 was transformed to give 788 colonies. The results of analysis of 16 clones out of 788 colonies digested with restriction enzyme reveal that 7 clones contained the desired plasmid. Thus, 223 µg of plasmid pTRSNGF having the signal sequence and the DNA sequence of the full length hNGF gene could be obtained.

E. coli strain HB101 was transformed with plasmid pTRSNGF to produce a transformant E. coli HB101 [pTRSNGF] which was deposited in the Fermenation Research Institute of the Agency of Industrial Science & Technology of Japan (FRI) on February 19, 1990 and given Accession Number P-11285, and then transferred to an international deposition under the Budapest Treaty on December 3, 1990 and given Accession Number BP-03187.

### Example 2

### Preparation of a plasmid wherein the gene encoding the N terminus of trpL polypeptide in tryptophan operon ligated with tryptophan regulatory gene is ligated upstream of the full length hNGF gene (cf. Fig. 9)

After 1 µg of plasmid pTRLBT1 was digested with 15 U of Eco RI and 20 U of Bam HI, 0.8% agarose gel electrophoresis was performed to recover the DNA fragment of the vector. On the other hand, 10 µg of plasmid pTRSNGFA was digested similarly with 75 U of Eco RI and 50 U of Bam HI followed by 5% polyacrylamide gel electrophoresis. Thus, the DNA fragment containing a part of DNA sequence A of 146 bp was recovered. After 1 µl each of these two DNA fragments were ligated using the ligation kit, E. coli strain C600 was transformed with the ligation product, whereby 143 colonies were obtained. Among them, DNAs of 8 clones were digested with restriction enzyme for analysis. The results reveal that 7 clones contained the desired plasmid. Thus, 10 µg of plasmid pTRLNGFA′ was obtained.

Next, 1 µg of this plasmid pTRLNGFA′ was digested with 15 U of Eco RI and 0.8% agarose gel electrophoresis was then performed to recover the DNA fragment of the vector. On the other hand, 500 pmols each of the chemically synthesized two linker DNAs (5'-AATTCAGCTCTTCCCACCCGATTTTCCACCGTGGCG-3' and 5'-AATTCGCCACGGTGGAAAATCGGGTGGGAAGAGCTG-3' were mixed with each other. After keeping at 75°C for 10 minutes, the mixture was cooled over 2 hours to effect annealing. Thus, double stranded DNA was prepared. The 5' end of this double stranded DNA was phosphorylated using 10 U of T4 polynucleotide kinase. After 1 µl each of the vector DNA and linker DNA were ligated using the ligation kit, E. coli strain C600 was transformed to give 6 colonies. Among them, DNAs of 6 clones were digested with restriction enzyme for analysis. The results reveal that 1 clone contained the desired plasmid. Thus, 29 µg of plasmid pTRLNGFA was obtained.

Next, 2 µg of plasmid pTRLNGFA was digested with 30 U of Nsp (7524) V and 50 U of Bam HI, and 0.8% agarose gel electrophoresis was then performed to recover the DNA fragment containing the vector and DNA sequence A. On the other hand, 2 µg of plasmid pTRLNGFB was digested with 30 U of Nsp (7524) V and 50 U of Bam HI, and 5% polyacrylamide gel electrophoresis was then performed to recover the DNA fragment containing DNA sequence B of 199 bp. After 0.5 µl each of these two fragments were ligated using the ligation kit, E. coli strain C600 was transformed to give 224 colonies. Among them, DNAs of 6 clones were digested with restriction enzyme for analysis. The results reveal that 2 clones contained the desired plasmid. Thus, 110 µg of plasmid pTRLNGF having the entire DNA sequence of the hNGF gene ligated with the gene encoding the N terminus of trpL polypeptide could be obtained.

E. coli strain HB101 was transformed with plasmid pTRLNGF to produce a transformant E. coli HB101 [pTRLNGF] which was deposited in FRI on February 19, 1990 and given Accession Number P-11283, and then transferred to an international deposition under the Budapest Treaty on December 3, 1990 and given Accession Number BP-03185.

### Example 3

### Production of hNGF by transformed microorganism not using tryptophan

E. coli HB101 [pTRSNGF], and E. coli HB101 [pTRLNGF] were cultured to produce hNGF, respectively, as described below.

One platinum loop of the stock strain was inoculated on 10 ml of M9 medium (1 g of NH₄Cl, 6 g of Na₂HPO₄, 3 g of KH₂PO₄, 0.5 g of NaCl, 0.015 g of CaCl₂.2H₂O, 0.5 g of MgSO₄.7H₂O, 2.5 g of Casamino acid, 5 g of glucose, 1.5 g of yeast extract, 0.04 g of tryptophan, 0.1 g of proline, 0.1 g of thiamine, 50 mg of ampicillin, 1 liter of water; pH 7.4) followed by culturing at 37°C overnight. The preincubated solution, 5 ml, was taken and centrifuged at 15000 rpm for a minute to recover the cells. Then, the cells were washed with 1 ml of modified M9 medium which was free from Trp and from which yeast extract and tryptophan were removed. The cells were inoculated on 50 ml of the modified M9 medium followed by incubation at 37°C for 8 hours. The cells after incubation for 8 hours were recovered by centrifugation. After washing with 50 mM Tris-HCl buffer (pH 7.5), the cells were resuspended in pure water to show absorbance about 10 at 550 nm. After 50 µl of sample buffer (0.25 M Tris-HCl, pH 6.8; 8% SDS; 40% glycerol; 20% β-mercaptoethanol; 0.004% BPB) and 5 µl of 50% glycerol BPB solution were added to 150 µl of this suspension, the mixture was heated at 100°C for 5 minutes. After 40 µl of this solution was subjected to 14% polyacrylamide gel electrophoresis, the gel was stained with Coomassie Brilliant Blue R-250 so that a band expected to be hNGF was observed around standard βNGF. The protein in the gel was transferred onto a nitrocellulose membrane and hNGF was detected with polyclonal antibody to 2.5 SNGF of mouse submaxillary gland. Binding with the antibody was observed at almost the same position as the stained band. Thus, expression of hNGF by E. coli could be confirmed. An amount of hNGF produced by E. coli was calculated based on the density of the color in the stained band. In E. coli HB101 [pTRLNGF], and E. coli HB101 [pTRSNGF] about 20%, about 3% of hNGF were produced, respectively, based on the amount of protein in the cells. Example 4

### Production of hNGF by transformed microorganism using IA

E. coli HB101 [pTRSNGF[, and E. coli HB101 (pTRLNGF) were cultured as described below, respectively, to produce hNGF.

In a manner similar to Example 3, 50 ml of the cells were inoculated on 50 ml of the modified M9 medium followed by culturing at 37°C for 8 hours. In order to express the gene, 15 µg/ml of IA was supplemented one hour after the culture. After completion of the culture, the system was treated as in Example 3. An amount of hNGF produced by the recombinant E. coli was determined. In E. coli HB101 [pTRLNGF], and E. coli HB101 [pTRSNGF], about 25%, about 4% of hNGF, which were almost the same as in Example 3, were produced, respectively, based on the amount of protein in the cells. the cells.

While the invention has been described in detail and with reference to specific embodiments thereof, it will be apparent to one skilled in the art that various changes and modifications can be made therein without departing from the spirit and scope thereof.

## Claims

1. DNA encoding human nerve growth factor and having the following nucleotide sequence: 
A G C T C T T C C C A C C C G A T T T T C
C A C C G T G G C G A A T T C T C C G T G
T G T G A C T C T G T C T C T G T A T G G
G T A G G C G A T A A A A C C A C T G C C
A C T G A T A T C A A A G G C A A A G A G
G T G A T G G T G C T G G G C G A A G T T
A A C A T T A A C A A C T C T G T A T T C
A A A C A G T A C T T C T T C G A A A C C
A A A T G C C G T G A C C C A A A C C C G
G T T G A C T C T G G C T G C C G C G G C
A T C G A T T C T A A A C A C T G G A A C
T C T T A C T G C A C C A C T A C T C A C
A C C T T C G T A A A A G C T T T G A C T
A T G G A T G G T A A A C A G G C T G C C
T G G C G T T T C A T C C G T A T C G A T
A C T G C A T G C G T G T G T G T A C T G
T C C C G T A A A G C T G T T C G T
 wherein DNA encoding the signal sequence of β-lactamase is ligated upstream of the 5' end of said DNA encoding human nerve growth factor.

2. DNA according to claim 1, wherein said DNA encoding the signal sequence of β-lactamase has the following nucleotide sequence:

A T G A G T A T T C A A C A T T T C C G T
G T C G C C C T T A T T C C C T T T T T T
G C G G C A T T T T G C C T T C C T G T T
T T T G C G

3. DNA according to claim 1 or 2, wherein further tryptophan regulatory gene is ligated upstream of said signal sequence.

4. DNA encoding human nerve growth factor and having the following nucleotide sequence: 
A G C T C T T C C C A C C C G A T T T T C
C A C C G T G G C G A A T T C T C C G T G
T G T G A C T C T G T C T C T G T A T G G
G T A G G C G A T A A A A C C A C T G C C
A C T G A T A T C A A A G G C A A A G A G
G T G A T G G T G C T G G G C G A A G T T
A A C A T T A A C A A C T C T G T A T T C
A A A C A G T A C T T C T T C G A A A C C
A A A T G C C G T G A C C C A A A C C C G
G T T G A C T C T G G C T G C C G C G G C
A T C G A T T C T A A A C A C T G G A A C
T C T T A C T G C A C C A C T A C T C A C
A C C T T C G T A A A A G C T T T G A C T
A T G G A T G G T A A A C A G G C T G C C
T G G C G T T T C A T C C G T A T C G A T
A C T G C A T G C G T G T G T G T A C T G
T C C C G T A A A G C T G T T C G T
 wherein DNA encoding the N terminus of trpL polypeptide in tryptophan operon ligated with a tryptophan regulatory gene is ligated upstream of the 5' end of said DNA encoding human nerve growth factor.

5. DNA according to claim 4, wherein said DNA encoding the N terminus of trpL polypeptide in tryptophan operon has the following nucleotide sequence:

A T G A A A G C A A T T T C G T G G A A
T T C

6. A plasmid vector carrying DNA according to any one of claims 1 to 5.

7. A transformant obtained by transforming a microorganism with a plasmid vector according to claim 6.

8. A transformant according to claim 7,
wherein the microorganism is E. coli.

9. A process for producing human nerve growth factor which comprises culturing a transformant according to claim 7 or 8 to produce human nerve growth factor.

10. A process for producing human nerve growth factor according to claim 9, wherein the transformant is cultured in the presence of 3-β-indolylacrylic acid (IA) as an expression inducer.

11. A process for producing human nerve growth factor according to claim 9 or 10, wherein the transformant is cultured in medium free of tryptophan.

## Patentansprüche

1. Menschlichen Nervenwachstumsfaktor codierende DNA mit der folgenden Nucleotidsequenz: 
A G C T C T T C C C A C C C G A T T T T C
C A C C G T G G C G A A T T C T C C G T G
T G T G A C T C T G T C T C T G T A T G G
G T A G G C G A T A A A A C C A C T G C C
A C T G A T A T C A A A G G C A A A G A G
G T G A T G G T G C T G G G C G A A G T T
A A C A T T A A C A A C T C T G T A T T C
A A A C A G T A C T T C T T C G A A A C C
A A A T G C C G T G A C C C A A A C C C G
G T T G A C T C T G G C T G C C G C G G C
A T C G A T T C T A A A C A C T G G A A C
T C T T A C T G C A C C A C T A C T C A C
A C C T T C G T A A A A G C T T T G A C T
A T G G A T G G T A A A C A G G C T G C C
T G G C G T T T C A T C C G T A T C G A T
A C T G C A T G C G T G T G T G T A C T G
T C C C G T A A A G C T G T T C G T
 wobei DNA, welche die Signalsequenz von β-Lactamase codiert, oberhalb des 5'-Endes der menschlichen Nervenwachstumsfaktor codierenden DNA eingebunden ist.

2. DNA nach Anspruch 1, wobei die die Signalsequenz von β-Lactamase codierende DNA die folgende Nucleotidsequenz besitzt:

A T G A G T A T T C A A C A T T T C C G T
G T C G C C C T T A T T C C C T T T T T T
G C G G C A T T T T G C C T T C C T G T T
T T T G C G

3. DNA nach Anspruch 1 oder 2, wobei ein weiteres, Tryptophan regulierendes Gen oberhalb der Signalsequenz eingebunden ist.

4. Menschlichen Nervenwachstumsfaktor codierende DNA mit der folgenden Nucleotidsequenz: 
A G C T C T T C C C A C C C G A T T T T C
C A C C G T G G C G A A T T C T C C G T G
T G T G A C T C T G T C T C T G T A T G G
G T A G G C G A T A A A A C C A C T G C C
A C T G A T A T C A A A G G C A A A G A G
G T G A T G G T G C T G G G C G A A G T T
A A C A T T A A C A A C T C T G T A T T C
A A A C A G T A C T T C T T C G A A A C C
A A A T G C C G T G A C C C A A A C C C G
G T T G A C T C T G G C T G C C G C G G C
A T C G A T T C T A A A C A C T G G A A C
T C T T A C T G C A C C A C T A C T C A C
A C C T T C G T A A A A G C T T T G A C T
A T G G A T G G T A A A C A G G C T G C C
T G G C G T T T C A T C C G T A T C G A T
A C T G C A T G C G T G T G T G T A C T G
T C C C G T A A A G C T G T T C G T
 wobei DNA, welche den N-Terminus von trpL-Polypeptid in einem mit einem Tryptophan regulierenden Gen verknüpften Tryptophan-Operon codiert, oberhalb des 5'-Endes der menschlichen Nervenwachstumsfaktor codierenden DNA eingebunden ist.

5. DNA nach Anspruch 4, wobei die DNA, welche den N-Terminus von trpL-Polypeptid in einem Tryptophan-operon codiert, die folgende Nucleotidsequenz besitzt:

A T G A A A G C A A T T T C G T G G A A
T T C

6. Plasmidvektor als Träger für DNA nach einem der Ansprüche 1 bis 5.

7. Transformante, die gewonnen wurde durch Transformation eines Mikroorganismus mit einem Plasmidvektor nach Anspruch 6.

8. Transformante nach Anspruch 7, wobei der Mikroorganismus E. coli ist.

9. Verfahren zur Herstellung von menschlichem Nervenwachstumsfaktor, bei dem eine Transformante nach Anspruch 7 oder 8 kultiviert wird, um menschlichen Nervenwachstumsfaktor zu produzieren.

10. Verfahren zur Herstellung von menschlichem Nervenwachstumsfaktor nach Anspruch 9, bei dem die Transformante in Gegenwart von 3-β-Indolylacrylsäure (IA) als Expressionsinduktor kultiviert wird.

11. Verfahren zur Herstellung von menschlichem Nervenwachstumsfaktor nach Anspruch 9 oder 10, bei dem die Transformante in einem Medium ohne Tryptophan kultiviert wird.

## Revendications

1. ADN codant le facteur de croissance des nerfs humain et ayant la séquence nucléotidique suivante : 
A G C T C T T C C C A C C C G A T T T T C
C A C C G T G G C G A A T T C T C C G T G
T G T G A C T C T G T C T C T G T A T G G
G T A G G C G A T A A A A C C A C T G C C
A C T G A T A T C A A A G G C A A A G A G
G T G A T G G T G C T G G G C G A A G T T
A A C A T T A A C A A C T C T G T A T T C
A A A C A G T A C T T C T T C G A A A C C
A A A T G C C G T G A C C C A A A C C C G
G T T G A C T C T G G C T G C C G C G G C
A T C G A T T C T A A A C A C T G G A A C
T C T T A C T G C A C C A C T A C T C A C
A C C T T C G T A A A A G C T T T G A C T
A T G G A T G G T A A A C A G G C T G C C
T G G C G T T T C A T C C G T A T C G A T
A C T G C A T G C G T G T G T G T A C T G
T C C C G T A A A G C T G T T C G T
 dans lequel un ADN codant la séquence signal de la β-lactamase est lié en amont de l'extrémité 5' dudit ADN codant le facteur de croissance des nerfs humain.

2. ADN selon la revendication 1, dans lequel ledit ADN codant la séquence signal de la β-lactamase à la séquence nucléotidique suivante :

A T G A G T A T T C A A C A T T T C C G T
G T C G C C C T T A T T C C C T T T T T T
G C G G C A T T T T G C C T T C C T G T T
T T T G C G

3. ADN selon la revendication 1 ou 2, dans lequel en outre un gène régulateur du tryptophane est lié en amont de ladite séquence signal.

4. ADN codant le facteur de croissance des nerfs humain et ayant la séquence nucléotidique suivante : 
A G C T C T T C C C A C C C G A T T T T C
C A C C G T G G C G A A T T C T C C G T G
T G T G A C T C T G T C T C T G T A T G G
G T A G G C G A T A A A A C C A C T G C C
A C T G A T A T C A A A G G C A A A G A G
G T G A T G G T G C T G G C G A A G T T
A A C A T T A A C A A C T C T G T A T T C
A A A C A G T A C T T C T T C G A A A C C
A A A T G C C G T G A C C C A A A C C C G
G T T G A C T C T G G C T G C C G C G G C
A T C G A T T C T A A A C A C T G G A A C
T C T T A C T G C A C C A C T A C T C A C
A C C T T C G T A A A A G C T T T G A C T
A T G G A T G G T A A A C A G G C T G C C
T G G C G T T T C A T C C G T A T C G A T
A C T G C A T G C G T G T G T G T A C T G
T C C C G T A A A G C T G T T C G T
 dans lequel un ADN codant l'extrémité N-terminale du polypeptide trpL dans l'opéron tryptophane lié à un gène régulateur du tryptophane est lié en amont de l'extrémité 5' dudit ADN codant le facteur de croissance des nerfs humain.

5. ADN selon la revendication 4, dans lequel ledit ADN codant l'extrémité N-terminale du polypeptide trpL dans l'opéron tryptophane à la séquence nucléotidique suivante :

A T G A A A G C A A T T T T C G T G G A A
T T C

6. Vecteur plasmidique portant un ADN selon l'une quelconque des revendications 1 à 5.

7. Transformant obtenu par transformation d'un micro-organisme avec un vecteur plasmidique selon la revendication 6.

8. Transformant selon la revendication 7, dans lequel le micro-organisme est E. coli.

9. Procédé pour la production du facteur de croissance des nerfs humain, qui comprend la culture d'un transformant selon la revendication 7 ou 8 pour produire le facteur de croissance des nerfs humain

10. Procédé pour la production du facteur de croissance des nerfs humain selon la revendication 9, dans lequel le transformant est cultivé en présence d'acide 3-β-indolylacrylique (IA) comme inducteur de l'expression.

11. Procédé pour la production de croissance des nerfs humain selon la revendication 9 ou 10, dans lequel le transformant est cultivé dans du milieu dépourvu de tryptophane.
